(19) Europäisches Patentamt
European Patent Office
Office européen des brevets

(11) Veröffentlichungsnummer : **0 093 425**
**B1**

(12) **EUROPÄISCHE PATENTSCHRIFT**

(45) Veröffentlichungstag der Patentschrift : **17.07.85**

(21) Anmeldenummer : **83104214.8**

(22) Anmeldetag : **29.04.83**

(51) Int. Cl.⁴ : **C 07 C 49/21**, C 07 C 47/225, C 07 C 45/74, C 11 B 9/00, A 61 K 7/46

(54) **Ungesättigte, cyclische Ketone.**

(30) Priorität : **03.05.82 US 374368**

(43) Veröffentlichungstag der Anmeldung : **09.11.83 Patentblatt 83/45**

(45) Bekanntmachung des Hinweises auf die Patenterteilung : **17.07.85 Patentblatt 85/29**

(84) Benannte Vertragsstaaten : **CH DE FR GB LI NL**

(56) Entgegenhaltungen :
**DE-A- 2 601 144**
**FR-A- 1 204 407**
**US-A- 2 815 378**
**US-A- 4 173 585**
**CHEMICAL ABSTRACTS, vol. 93, 1980, page 691, no. 149961s, Columbus, Ohio, USA**

(73) Patentinhaber : **L. GIVAUDAN & CIE Société Anonyme**
**CH-1214 Vernier-Genève (CH)**

(72) Erfinder : **Virgilio, Joseph A.**
**14 Evelyn Terrace**
**Wayne, N.J. 07470 (US)**
Erfinder : **Hellwell, Emanuel**
**2 Birch Tree Drive**
**Fairfield, N.J. 07006 (US)**

(74) Vertreter : **Urech, Peter, Dr. et al**
**Grenzacherstrasse 124 Postfach 3255**
**CH-4002 Basel (CH)**

**EP 0 093 425 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft neue Ketone der Formel

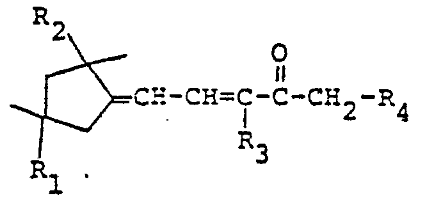

(I)

worin $R_1$, $R_2$, $R_3$ und $R_4$ Wasserstoff oder Methyl darstellen, wobei $R_1 \neq R_2$ und $R_3 \neq R_4$ sind.

Die Verbindungen der Formel I verfügen über wertvolle Holznoten, es handelt sich demgemäss im Falle dieser Verbindungen um wertvolle Ingredienzien von Riechstoffkompositionen.

Die Erfindung betrifft auch Riechstoffkompositionen mit einem Gehalt an den neuen Ketonen I.

Die Erfindung betrifft auch eine Verfahren zur Herstellung der neuen Verbindungen I. Dieses Verfahren ist dadurch gekennzeichnet, dass man einen Aldehyd IV (siehe unten) mit 2-Butanon umsetzt. Dieses Verfahren und die Herstellung der neuen Ausgangsmaterialien IV können wie folgt dargestellt werden :

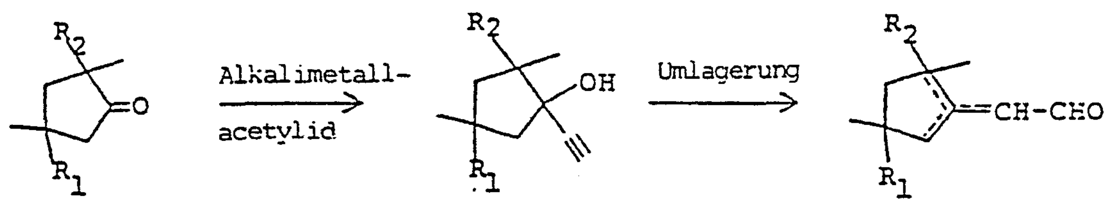


(II) (III) (IV)

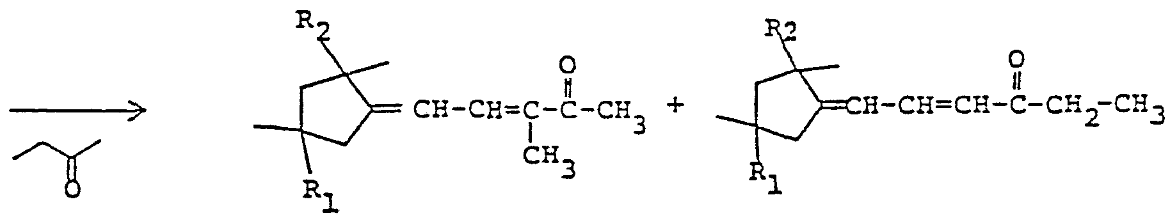

(Ia) (Ib)

Die Umsetzung des Aldehyd IV mit 2-Butanon liefert üblicherweise ein Gemisch der Isomeren Ia und Ib ; es handelt sich also um die Verbindungen der Formel I, worin $R_3$ Methyl und $R_4$ Wasserstoff darstellen (Verbindungen Ia) bzw. um Verbindungen worin $R_3$ Wasserstoff und $R_4$ Methyl darstellen (Verbindungen der Formel Ib).

Das Ausgangsmaterial II wird zweckmässigerweise gemäss dem oben stehenden Schema hergestellt :

Bei der Reaktion des Trimethylcyclopentanons II mit dem Alkalimetallacetylid zum Aethinylalkohol III kann das Ausgangsketon als reines Isomer IIa ($R_1$ = Wasserstoff) oder als reines Isomer IIb ($R_2$ = Wasserstoff) oder als Gemisch dieser Verbindungen eingesetzt werden. Aus ökonomischen Gründen wird vorzugsweise das Handelsprodukt eingesetzt, bei welchem Produkt es sich um ein Gemisch mit ungefähr 60 % IIa und 40 % IIb handelt. Die beiden Verbindungen IIa und IIb können aber auch in reiner Form hergestellt werden, z. B. gemäss J. Org. Chem. *25* 514 (1960). Das reine IIb kann schliesslich gemäss Org. Syn. *39*, 70 (1959) und Org. Syn. *37*, 58 (1957) rein dargestellt werden.

Das Keton II kann durch Umsetzung mit einem Alkalimetallacetylid in den Aethinylalkohol III übergeführt werden. Die Methodik, um Ketone mittels, Alkalimetallacetyliden in Aethinylalkohole überzuführen, ist bekannt, siehe diesbezüglich beispielsweise Heinz Günter Viehe, « Chemistry of Acetylenes » Kapitel 3, Marcel Dekker, New York, 1969 : hier werden eine Reihe solcher in Frage kommende Methoden abgehandelt. Vorzugsweise wird man zu den Methoden greifen, die von Natriumacetylid in Ammoniak bzw. dem Lithiumacetylid/Aethylendiaminkomplex, KOH und Acetylen in einem organischen Lösungsmittel Gebrauch machen.

Die Umlagerung von Aethinylalkoholen in ungesättigte Aldehyde ist ebenfalls bekannt. Zu erwähnen sind hier beispielsweise sauerkatalysierte Umlagerungen, z. B. die Meyer-Schuster-Umlagerung, wobei

aber oft Produkte von komplexer Zusammensetzung anfallen. Bessere Ausbeuten werden erzielt, wenn die esterifizierten Aethynylalkohole in Anwesenheit gewisser Uebergangsmetalle umgelagert werden, oder wenn die Aethinylalkohole in Anwesenheit von silylierten Vanadaten umgelagert werden.

Vorzugsweise arbeitet man gemäss Pauling et al., Helv. Chem. Acta, *59*, 1233 (1976), d. h. unter Zuhilfenahme eines tris-Trialkylsilyl- oder Triarylsilylvanadates, wobei das tris-(Triphenylsilylvanadat) speziell bevorzugt ist. Das bevorzugte Verhältnis von Katalysator zu Aethinylalkohol beträgt ungefähr 0,01 bis 0,1 Mol/Mol, wobei ungefähr 0,03 bis 0,04 besonders bevorzugt sind. Kohlenwasserstoffe, Aether und Mineralöle sind geeignete Lösungsmittel. Temperaturen zwischen 100 und 150° sind bevorzugt, besonders bevorzugt ist der Bereich von 130 bis 145°. Vorzugsweise arbeitet man ferner unter Zuhilfenahme eines Ueberschusses an Triphenylsilanol und einer Carbonsäure, beispielsweise Benzoesäure, um Dehydratisierung zu verhindern.

Die Kondensation des Aldehydes IV mit 2-Butanon kann gemäss den in der Literatur für die Kondensation von Ketonen mit Aldehyden beschriebenen Methoden durchgeführt werden. Es handelt sich hierbei also um eine Reaktion vom Aldoltyp. Es wird demgemäss vorzugsweise unter Verwendung einer starken Base, beispielsweise wässrig-methanolischen Kaliumhydroxid und unter Verwendung eines Ueberschusses an dem billigeren Keton gearbeitet. Vorzugsweise arbeitet man bei − 15 bis 5 °C, Reaktionszeit zwischen 10 bis 48 Stunden, gefolgt von einer Reaktionszeit derselben Grössenordnung bei 15 bis 40 °C. Speziell bevorzugt ist eine Reaktionszeit von 20 bis 28 Stunden bei Temperaturen von − 15 bis 5 °C, gefolgt von derselben Reaktionszeit bei Temperaturen von 25 bis 30 °C. Das Reaktionsprodukt stellt ein Isomerengemisch der Ketone I dar, wobei es sich üblicherweise um 70 bis 90 % der Struktur Ia handelt, nämlich um 3-Methyl-1(2,2,4- und 2,4,4-Trimethyl-1-cyclopentyliden)pent-2-en-4-on und um 30 bis 10 % der Struktur Ib, nämlich um 1-(2,2,4- und 2,4,4-Trimethyl-1-cyclopentyliden)hex-2-en-4-on. Das Keton Ia kann durch Destillation, beispielsweise durch eine Drehbandkolonne, vom Keton Ib abgetrennt werden.

Das reine Ia verfügt über einen holzigen, jononartigen und fruchtigen Geruch. Die Verbindung ist insbesondere zur Einarbeitung in wertvolle Edelholzbasen geeignet, Beispiele sind Vetiver, Sandelholz, etc., in welchen Kompositionen der Edelholzcharakter verbessert wird, und der Komposition gleichzeitig Körper und Fülle verliehen wird.

Vorzugsweise wird das Reaktionsgemisch, das nach dem erfindungsgemässen Verfahren anfällt, verwendet, dieses enthält zwischen 10 und 30 % Ib, üblicherweise ca. 20 %. Dieses Gemisch weist dieselben wertvollen holzigen Riechstoffeigenschaften auf wie das reine Ia, wobei hier auch noch Olibanum- und Butternoten erwähnt werden müssen. Das Gemisch kann üblicherweise in allen Kompositionen verwendet werden, für welche das reine Ia geeignet ist. In gewissen Kompositionen wirkt sich sogar der zusätzliche würzige Charakter des Isomerengemisches positiv aus. Beispielsweise hat es sich gezeigt, dass bei Zugabe zu einer Ambrakomposition das Gemisch von 80 % Ia und 20 % Ib die Citrus-, tierischen und süssen Noten gut verbindet und dazu der ganzen Komposition eine erwünschte würzige Note verleiht ; es resultiert eine gut abgerundete, würzige Ambrakomposition.

Die neuen Ketone können in Riechstoffkompositionen beispielsweise von ca. 0,1 bis ca. 20 % — je nach Verwendungszweck — eingesetzt werden. Zur Erzielung spezieller Effekte können aber auch Konzentrationen von über 20 % angezeigt sein.

Die neuen Ketone können bei der Herstellung von Riechstoffkompositionen, die wiederum als Riechstoffbasen bei der Herstellung von Parfüms und Toilettenwässern verwendet werden, eingesetzt werden ; zur Herstellung der kosmetischen Verbrauchsgüter wird hierauf noch in üblicher Weise verdünnt, beispielsweise mit Alkohol und/oder Wasser : im Falle von Parfüms wird ungefähr 15-20 % der Base benötigt, im Falle der Toilettenwässer ungefähr 3-5 % der Base.

Die Basen können aber auch für Seifen, Detergenzien und anderen Kosmetika Verwendung finden. In diesen Fällen wird üblicherweise eine Basenkonzentration von ungefähr 0,5 bis ungefähr 2 Gewichtsprozent zum Einsatz gelangen.

Die folgenden Beispiele illustrieren die bevorzugten Aspekte der vorliegenden Erfindung. Sie sind rein illustrierend, sie sollen also nicht limitierend verstanden werden. Bei den Parfümingredienzien handelt es sich um Teile/1000, das Gewicht ist in Gramm angegeben. Die Analysen wurden wie folgt durchgeführt :

Die Massenspektren wurden auf einem Finnigan Modell 4 000 ausgeführt ; die Massenzahlen sind in m/e angegeben. Die Kernresonanzspektren wurden aufgenommen in Lösungen in $CDCl_3$ auf einem Varian Modell EM 360L Spektrometer, die Ergebnisse sind als δ-Einheiten relativ zu TMS (Tetramethylsilan mit 0,0 δ) angegeben. Die gaschromatographischen Analysen wurden auf einem Hewlett-Packard Modell 5880A unter Verwendung einer 0,25 mm × 12 m Carbowax 20M, $SiO_2$ Quarz-Kapillarkolonne durchgeführt. Die Infrarotspektren wurden auf einem Perkin-Elmer Modell 457 aufgenommen ; die Werte sind als $cm^{-1}$ wiedergegeben.

Beispiel 1

3-Methyl-1-(2,2,4- und 2,4,4-Trimethyl-1-cyclopentyliden)-pent-2-en-4-on, Ia

a) Ein Gemisch von 105 ml n-Butanol und 600 g pulverisiertem Kaliumhydroxid in 1,4 l trockenem

Toluol wurde unter Stickstoff 30 Minuten auf 100 °C erhitzt. Das Gemisch wurde auf – 5 °C abgekühlt, und es wurden 100 ml Dimethylformamid zugegeben. Acetylengas und eine Lösung von 2,2,4- (und 2,4,4-) Trimethylcyclopentanon (315 g) in 338 ml trockenem Toluol wurden während 2 Stunden simultan eingeführt. Die Acetylenzugabe wurde noch weitere 3 Stunden weitergeführt. Nach Verdrängen des nicht umgesetzten Acetylens mit Wasserstoff und Giessen des Reaktionsgemisches auf 1,3 l Wasser wurde die Toluolschicht abgetrennt, mit 500 ml 5 %-igem $NaHCO_3$ gewaschen und schliesslich zu einem Oel konzentriert, wobei die Ausbeute an letzterem 264 g betrug. Das Prozedere wurde 3 mal wiederholt, es fielen auf diese Weise nach Destillation 467 g Aethinylalkohol III an, Siedepunkt 87-90 °C/26 mm Hg.

b) Zu einem Gemisch von 47,5 g (0,17 M) Triphenylsilanol, 22,4 g (0,025 M) tris-Triphenylsilylvanadat und 2,2 g Benzoesäure in 1,587 l Mineralöl wurden 100 g (0,68 M) des Aethinylalkohols gemäss Ia) gegeben. Das Gemisch wurde 7 Stunden auf 140 °C erhitzt, darauf folgte eine Blitzdestillation des rohen Materials. Auf dieselbe Weise wurden 3 Destillate erstellt, wobei das Katalysatorsystem recycliert wurde. Bei 10 mm Hg wurde eine Totalmenge von 173 g des rohen Materials destilliert. Die Destillation wurde mittels Gaschromatographie verfolgt. Die Destillate mit einem Siedebereich von 80-102 °C wurden kombiniert und so 145 g des Produktes IV erhalten ; H-NMR, 10,09 δ und 9.80 (2d, α,β-unges. C = O), 9.43 bis 9.60 (multi, β,γ-unges. C=O).

c) Eine Lösung von 10,1 g Kaliumhydroxid, 123 g Wasser und 340 g Methanol wurde auf 5 °C abgekühlt. Dazu wurden 145,5 g 2-Butanon gegeben. Hierauf wurde der Aldehyd der obigen Stufe b) (50 g) innert 25 Minuten zugegeben, und zwar bei einer Temperatur von ± 5 °C. Das Gemisch wurde eine halbe Stunde gerührt, und hierauf 24 Stunden bei 0 bis 5 °C und darauf nochmals 24 Stunden bei 25 °C gehalten. Das Reaktionsgemisch wurde mit 12,3 g 62,5 %-iger Schwefelsäure angesäuert und der pH mittels 20 %-iger NaOH auf 6 gebracht. Die tiefsiedenden Komponenten wurden durch Destillation bei Atmosphärendruck bis zu einer Temperatur von 100 °C entfernt. Der Rückstand, er betrug 63,2 g, wurde neutralgewaschen und bei 2,0 mm Hg destilliert. Der Destillation folgte eine gaschromatographische Analyse. Die Fraktionen mit dem Siedeintervall von 113-118 °C wurden kombiniert ; aus diese Wiese wurden 38 g eines Produktes mit einem Gehalt von 96 % an zwei Komponenten im Verhältnis von 82 zu 18 erhalten. Die Identifikation geschah durch gaschromatographische Analyse und durch Massenspektralanalyse : vorhanden waren 2 Isomere, nämlich 3-Methyl-1-(2,2,4- und 2,4,4-Trimethyl-1-cyclopentyliden)pent-2-en-4-on (Ia) und 1-(2,2,4- und 2,4,4-Trimethyl-1-cyclopentyliden)hex-2-en-4-on (Ib) ; MS (m/e) Ia : (206) ; MG (163), Verlust von $CH_3C{=}O$ (43) $CH_3C{=}O$ ; Ib : (206) ; MG (149) Verlust von $CH_3CH_2C{=}O$ (57) $CH_3CH_2C{=}O$.

Geruch : holzig, Olibanum, butterig, Immortellen.

d) Das Produkt gemäss c) wurde durch eine Drehbandkolonnendestillation bei 0,9 mm Hg (Nester-Faust NFA 100 Auto-Annular Still) rektifiziert. Dabei konnte die Verbindung Ia chemisch rein erhalten werden. Siedepunkt 89-90 °C, MG 206 ; IR : 1670 (s), 1637 (s) ; H-NMR : 1.08 δ (3H, s) und 1.20 (3H, s, geminale Dimethylgruppe), 1.08 (3H, d, J = 5.5 Hz), 1.87 (3H, s, olefin. $CH_3$), 2.33 (3H, s, acetyl. $CH_3$), 6.13 (1H, d, J = 12) + t, (J-2), γ-H des Dienonsystems und 7.20 (1H, d, J = 12 Hz, β-H des Dienonsystems).

Geruch (Ia) : holzig, Jonon, fruchtig.

## Beispiel 2

### Vetiver-Base

| Komponenten | Gewichtsteile |
|---|---|
| Vetiveröl Bourbon | 290 |
| Santalol | 200 |
| Laurine® (Hydroxycitronellal) | 90 |
| Zimtalkohol | 50 |
| Heliotropin | 60 |
| Coumarin | 30 |
| Ketonmoschus (4-t-Butyl-3,5-dinitro-2,6-dimethylacetophenon) | 50 |
| Ambrettemoschus (6-t-Butyl-3-methyl-2,4-dinitroanisol) | 30 |
| Propylenglykol | 100 |
| | 900 |

Die Zugabe von 100 Teilen des Ketons Ia zu der obigen Vetiverbase verleiht der Komposition Körper und unterstreicht die Edelholznoten.

## Beispiel 3

### Amber-Base

| Komponenten | Gewichtsteile |
|---|---|
| Labdanumharz (lösl.) | 376,3 |

| | |
|---|---|
| Patchouliöl « Moyenne » (Gemisch versch. Provenienz) | 19,8 |
| Benzoinharz (lösl.) | 99,0 |
| Vetiveröl Bourbon | 49,5 |
| Sandelholzöl (ostind.) | 99,0 |
| Mousse de Chêne Absolue (50 % in Aethanol) | 19,8 |
| Ambrettemoschus (6-t-Butyl-3-methyl-2,4-dinitroanisol) | 49,5 |
| Ketonmoschus (4-t-Butyl-3,5-dinitro-2,6-dimethylacetophenon) | 49,5 |
| Vanillin | 49,5 |
| Bergamotteöl (Furocoumarin-frei) | 69,3 |
| Orangenöl (kalifornisch, kalt gepresst) | 49,5 |
| Rosenbase (hauptsächlich Phenyläthylalkohol, Citronellol, Phenylacetat) | 49,5 |
| Castoreum Absolue (10 % in Aethanol) | 9,9 |
| | 990,1 |

Die Zugabe von 9,9 Teilen des Ketongemisches I gemäss Beispiel 1c) zeigt eine beeindruckende Wirkung auf die obige Ambrabase. Ohne Zugabe von I würde die Citrus-, die tierische und die süsse Note nicht harmonieren. Zugabe von I bewirkt nun einen verbindenden Effekt und führt zugleich zu einem würzigen Charakter der Base, wodurch der Gesamteindruck dieser Base ausgesprochen positiv beeinflusst wird.

**Patentansprüche**

1. Verbindungen der Formel

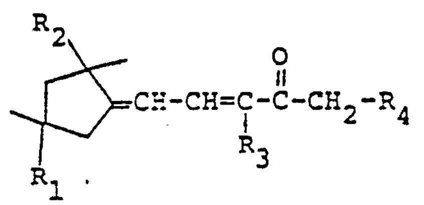

(I)

worin $R_1$, $R_2$, $R_3$ und $R_4$ Wasserstoff oder Methyl darstellen, wobei $R_1 \neq R_2$ und $R_3 \neq R_4$ sind.

2. Verbindungen gemäss Anspruch 1, worin $R_4$ Wasserstoff darstellt.

3. Verbindungen gemäss Anspruch 2, worin $R_2$ Wasserstoff darstellt.

4. Verbindung gemäss Anspruch 2, worin $R_2$ Methyl darstellt.

5. Isomerengemisch nach Anspruch 1, gekennzeichnet durch einen Gehalt an 90 bis 70 % an Derivaten mit $R_4$ = Wasserstoff und 10-30 % an Derivaten mit $R_4$ = Methyl.

6. Verbindungen der Formel

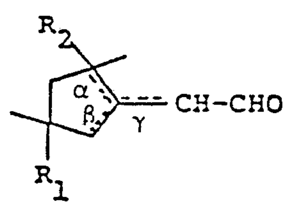

(IV)

worin eine der Gruppen $R_1$ oder $R_2$ Methyl und die andere Wasserstoff darstellt, und worin eine der gestrichelt gezeichneten Linien $\alpha$, $\beta$ oder $\gamma$ eine zusätzliche Bindung darstellt.

7. Verbindungen gemäss Anspruch 6, worin die zusätzliche Bindung in Stellung $\gamma$ ist.

8. Verbindungen der Formel

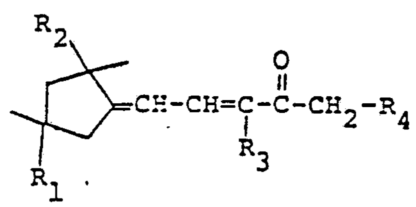

(I)

worin $R_1$, $R_2$, $R_3$ und $R_4$ Wasserstoff oder Methyl darstellen, wobei $R_1 \neq R_2$ und $R_3 \neq R_4$ sind, als Riechstoffe.

9. Riechstoffkomposition gekennzeichnet durch einen Gehalt an einer Verbindung der Formel

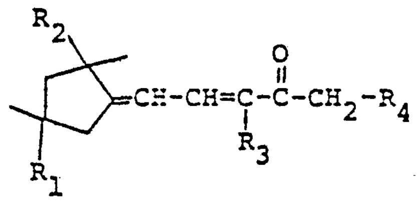

(I)

worin $R_1$, $R_2$, $R_3$ und $R_4$ Wasserstoff oder Methyl darstellen wobei $R_1 \neq R_2$ und $R_3 \neq R_4$ sind.

10. Komposition gemäss Anspruch 9, worin $R_4$ Wasserstoff darstellt.

11. Riechstoffkomposition, gekennzeichnet durch einen Gehalt an 90 bis 70 % an Derivaten mit $R_4$ = Wasserstoff und 10-30 % an Derivaten mit $R_4$ = Methyl.

12. Verfahren zur Herstellung von Verbindungen der Formel

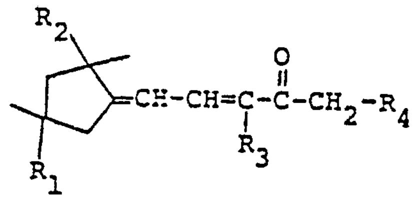

(I)

worin $R_1$, $R_2$, $R_3$ und $R_4$ Wasserstoff oder Methyl darstellen, wobei $R_1 \neq R_2$ und $R_3 \neq R_4$ sind, dadurch gekennzeichnet, dass man einen Aldehyd der Formel

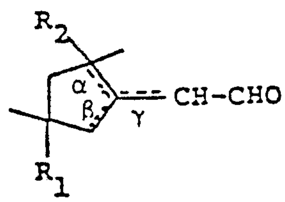

(IV)

worin $R_1$ und $R_2$ obige Bedeutung besitzen und eine der gestrichelt gezeichneten Linien eine zusätzliche Bindung darstellt,
mit 2-Butanon umsetzt.

13. Verwendung der Verbindungen der Formel

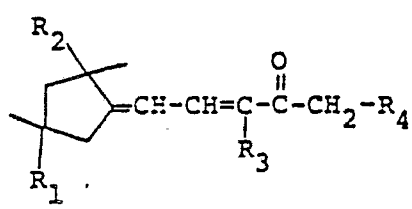

(I)

worin $R_1$, $R_2$, $R_3$ und $R_4$ Wasserstoff oder Methyl darstellen, wobei $R_1 \neq R_2$ und $R_3 \neq R_4$ sind, als Riechstoffe.

**Claims**

1. Compounds of the formula

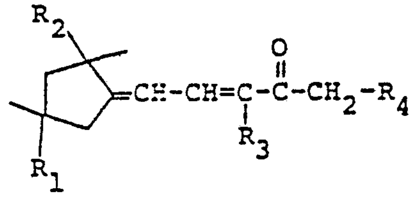

(I)

wherein $R_1$, $R_2$, $R_3$ and $R_4$ represent hydrogen or methyl, whereby $R_1 \neq R_2$ and $R_3 \neq R_4$.

2. Compounds in accordance with claim 1, wherein $R_4$ represents hydrogen.

3. Compounds in accordance with claim 2, wherein $R_2$ represents hydrogen.

4. The compound in accordance with claim 2, wherein $R_2$ represents methyl.

5. An isomer mixture according to claim 1, characterized by a content of 90 to 70 % of derivatives with $R_4$ = hydrogen and 10-30 % of derivatives with $R_4$ = methyl.

6. Compounds of the formula

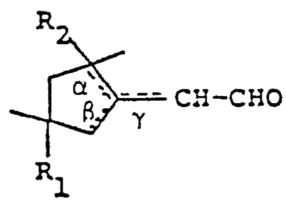

(IV)

wherein one of the groups $R_1$ or $R_2$ represents methyl and the other represents hydrogen, and wherein one of the lines α, β or γ shown dotwise represents an additional bond.

7. Compounds in accordance with claim 6, wherein the additional bond is in position γ.

8. Compounds of the formula

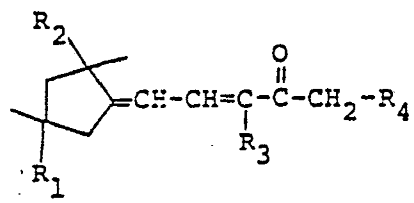

(I)

wherein $R_1$, $R_2$, $R_3$ and $R_4$ represent hydrogen or methyl, whereby $R_1 \neq R_2$ and $R_3 \neq R_4$, as odorant substances.

9. An odorant substance composition, characterized by a content of a compound of the formula

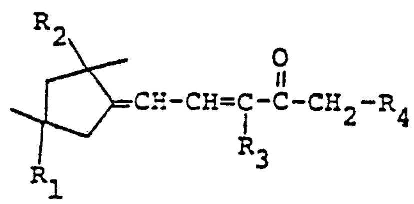

(I)

wherein $R_1$, $R_2$, $R_3$ and $R_4$ represent hydrogen or methyl, whereby $R_1 \neq R_2$ and $R_3 \neq R_4$.

10. A composition in accordance with claim 9, wherein $R_4$ represents hydrogen.

11. An odorant substance composition, characterized by a content of 90 to 70 % of derivatives with $R_4$ = hydrogen and 10-30 % of derivatives with $R_4$ = methyl.

12. A process for the manufacture of compounds of the formula

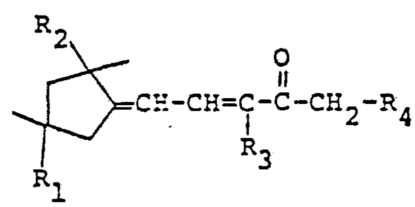

(I)

wherein $R_1$, $R_2$, $R_3$ and $R_4$ represent hydrogen or methyl, whereby $R_1 \neq R_2$ and $R_3 \neq R_4$, characterized by reacting an aldehyde of the formula

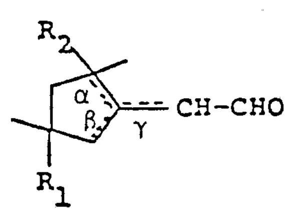

(IV)

wherein $R_1$ and $R_2$ have the above significance and one of the lines shown dotwise represents an additional bond,
with 2-butanone.

13. The use of the compounds of the formula

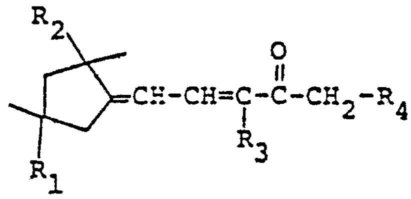

(I)

wherein $R_1$, $R_2$, $R_3$ and $R_4$ represent hydrogen or methyl, whereby $R_1 \neq R_2$ and $R_3 \neq R_4$, as odorant substances.

**Revendications**

1. Composés de formule

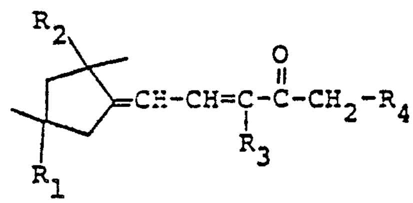

(I)

où $R_1$, $R_2$, $R_3$ et $R_4$ représentent un hydrogène ou un méthyle, où $R_1 \neq R_2$ et $R_3 \neq R_4$.

2. Composés selon la revendication 1, où $R_4$ représente un hydrogène.

3. Composés selon la revendication 2, où $R_2$ représente un hydrogène.

4. Composés selon la revendication 2, où $R_2$ représente un méthyle.

5. Mélange d'isomères selon la revendication 1, caractérisé en ce qu'il contient de 90 à 70 % de dérivés avec $R_4$ = hydrogène et de 10 à 30 % de dérivés avec $R_4 \neq$ méthyle.

6. Composés de formule

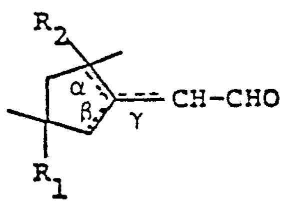

(IV)

où l'un des groupes $R_1$ ou $R_2$ représente un méthyle et l'autre un hydrogène, et où l'une des lignes $\alpha$, $\beta$ ou $\gamma$ représentées en pointillé représente une liaison supplémentaire.

7. Composés selon la revendication 6, où la liaison supplémentaire est en position $\gamma$.

8. Composés de formule

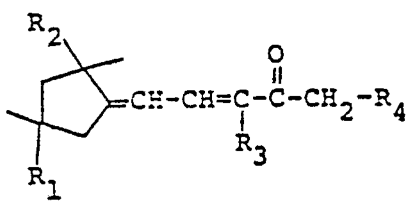

(I)

où $R_1$, $R_2$, $R_3$ et $R_4$ représentent un hydrogène ou un méthyle où $R_1 \neq R_2$ et $R_3 \neq R_4$, comme produits odorants.

9. Composition odorante caractérisée en ce qu'elle contient un composé de formule

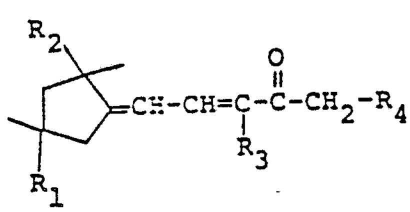

(I)

où $R_1$, $R_2$, $R_3$ et $R_4$ représentent un hydrogène ou un méthyle où $R_1 \neq R_2$ et $R_3 \neq R_4$.

10. Composition selon la revendication 9, où $R_4$ représente un hydrogène.

11. Composition odorante caractérisée en ce qu'elle contient de 90 à 70 % de dérivés avec $R_4$ = hydrogène et de 10 à 30 % de dérivés avec $R_4$ = méthyle.

12. Procédé de préparation de composés de formule

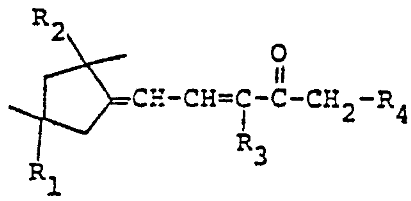

(I)

où $R_1$, $R_2$, $R_3$ et $R_4$ représentent un hydrogène ou un méthyle, où $R_1 \neq R_2$ et $R_3 \neq R_4$, caractérisé en ce qu'on fait réagir un aldéhyde de formule

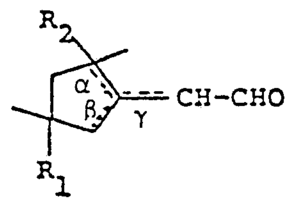

(IV)

où $R_1$ et $R_2$ ont la signification donnée ci-dessus et l'une des lignes représentées en pointillé représente une liaison supplémentaire, avec la 2-butanone.

13. Application des composés de formule

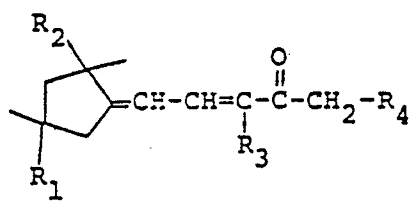

(I)

où $R_1$, $R_2$, $R_3$ et $R_4$ représentent un hydrogène ou un méthyle où $R_1 \neq R_2$ et $R_3 \neq R_4$, comme produits odorants.